# EUROPEAN PATENT SPECIFICATION

(11) **EP 3 320 346 B1**
(45) Date of publication and mention of the grant of the patent: **17.04.2019**
(21) Application number: 16750508.0
(22) Date of filing: 21.06.2016
(51) Int. Cl.: G01N 35/00, G07C 3/14

(54) **DEVICE AND METHOD FOR TESTING PACKAGED FOODSTUFFS FOR THE PRESENCE OF MICROORGANISMS**
VORRICHTUNG UND VERFAHREN ZUM TESTEN VON VERPACKTEN LEBENSMITTELN AUF ANWESENHEIT VON MIKROORGANISMEN
DISPOSITIF ET PROCÉDÉ POUR CONTRÔLER DES ALIMENTS EMBALLÉS DANS LE BUT DE DÉTECTER LA PRÉSENCE DE MICROORGANISMES

(30) Priority: 10.07.2015 NL 2015144
(43) Date of publication of application: 16.05.2018
(73) Proprietor: Amiris B.v., 6136 KT Sittard (NL)
(72) Inventor: RIJKX, Joseph Maria Franciscus Donatus, 6136 KT Sittard (NL); DIEPENBRUCK, Jens, 6136 KT Sittard (NL)
(74) Representative: Algemeen Octrooi- en Merkenbureau B.V.
(86) International application number: PCT/NL2016/050439
(87) International publication number: WO 2017/010867

(56) References cited:
- EP-A1- 1 588 945
- EP-A2- 0 330 495
- JP-A- H0 572 215
- US-A- 3 848 742

## Description

The invention relates to a device and a method for testing packaged foodstuffs such as milk or fruit juices, for example. The presence of microorganisms in such products can lead to premature decay of the packaged product.

In practice, packages containing foodstuffs such as milk or fruit juice packs, are filled in a production line. The end product will have to be subjected to a quality inspection. A number of samples will be taken at random from the products produced. In the meantime, the products produced that are related to the sample are placed in temporary storage. The samples are first placed in an incubation space so that any organisms that may be present in the product will grow to a detectable level. The incubation period depends on the product to be inspected. After the incubation period, the products are actually inspected and tested in a sample analysis device for the presence of microorganisms in the product. Depending on the outcome of the test, the products in temporary storage can be released for sale, further testing can take place, or the products in temporary storage can be declared unfit for consumption.

The present invention in particular relates to the part of the sample analysis device, i.e. the part of the actual inspection and testing for the presence of microorganisms in the number of samples selected or obtained from the production line, and consequently in particular to the part following the aforesaid incubation period up to and including the part of obtaining the test results.

According to the prior art, the packages that form part of the sample, are tested for the presence of microorganisms. A package, preferably in sealed condition, is supplied to the infeed unit. From the infeed unit, the packages move downstream to the sample analysis device. This sample analysis device comprises a probe which penetrates into the interior of the package for taking a sample of the liquid foodstuff. The sample is then placed in a sample container in the sample analysis device, which is configured to analyse any microorganisms that may be present in the sample, for example by adding reagents and carrying out a measurement on the sample for determining whether microorganisms are present, for example by means of an ATP microorganism measurement. The sample analysis device tests the sample, for example for the presence of ATP from microorganisms, while in the meantime the package from which the sample has been taken moves further downstream to a discharge unit. At the discharge unit, the packages can be removed from the device.

During the sample analysis period, the associated packages are stored, for example on a conveyor belt of the device or in a separate space. After the analysis of the sample, a result is linked to the package in question. The packages are to that end stored in a specific order, for example in the order in which the packages have been fed in. The stored packages wait for their results in a static condition. Depending on the result, the packages may be destroyed, and in the case of a positive value be tested otherwise for confirmation.

A drawback of the known method is the relatively large risk of an incorrect link between a package and the result. The result of a sample from the first package can be linked to another, second package, for example because the order of the stored packages does not correspond to the order of the results, or because a user makes a mistake in linking the result of the sample to the stored package. From document US 3,848,742 a glass container inspection machine is known, which comprises an infeed unit for a packaging sample, a sample analysis device which is disposed downstream of the infeed unit and which is configured for testing the sample, a discharge unit which is disposed downstream of the sample analysis device for discharging the package from the device, wherein the device comprises a storage unit for the package, which is disposed between the sample analysis device and the discharge unit, the device further comprises display means for displaying a result of the testing of the sample from the package at the location of the discharge unit, and wherein the device is configured so that the storage unit delivers the package at the discharge unit substantially at the same time as the results of the package in question are being displayed by the display means.

Accordingly it is an object of the present invention to provide an improved device for testing for the presence of microorganisms in packaged foodstuffs, wherein the risk of an incorrect link between a package and the result is reduced and preferably eliminated.

In order to achieve that object, the invention provides a device according to claim 1. The device according to the present invention comprises a storage unit for the package. This storage unit is disposed between the sample analysis device and the discharge unit, so that a package is transported downstream from the sample analysis device to the storage unit and from there to the discharge unit.

The device according to the present invention further comprises display means for displaying a result of the testing of the sample from the package. The results are displayed in such a manner that they are perceptible, preferably visible or audible, at the location of the discharge unit.

The device according to the present invention is further configured so that the storage unit delivers the package at the discharge unit substantially at the same time as the results of the package in question are being displayed by the display means. A package reaches the discharge unit at the same time as its respective results, as it were, so that the result is linked to the associated package in an effective manner. The present invention is based on the concept that if a package is perceived simultaneously with its result upon being discharged from the device, there can be no mix-up between results and packages. The object of the present invention is thus achieved.

The device is very suitable for testing for the presence of microorganisms in liquid foodstuffs such as milk or fruit juices.

The packages preferably arrive one by one at the discharge unit, so that the display of one result coincides with the arrival of one package so as to obtain a clear link between the package and the result. It is, incidentally, conceivable that parallel devices, each comprising a discharge unit provided with display means, are provided so as to obtain a quicker throughput of packages.

In one embodiment, the device comprises a control unit which is operatively connected to the sample analysis device, the storage unit, the discharge unit and the display means, and which is configured to control the device for testing the sample from the package substantially fully automatically, in such a manner that the storage unit delivers the package at the discharge unit substantially at the same time as the results of the package in question are being displayed. By using such a control unit, the user only needs to supply the package to be analysed to the infeed unit, after which testing of the package and displaying of the result upon discharge of the product will take place fully automatically, in dependence on the associated assay.

In one embodiment, the storage unit comprises a conveyor, for example a conveyor belt, conveyor rollers, trolleys or other known conveying means. The conveyor conveys the package through the storage unit to the discharge unit.

The conveyor is preferably configured to carry a multitude of packages into and out of the storage unit in the same order. The conveyor operates according to the "first in, first out" principle, so that the first package of a series of packages carried into the storage unit will also be the first package carried out of the storage unit. The sample analysis device preferably tests the samples in the same order as the packages are stored in a storage unit, so that the series of results can be linked to the series of packages in a simple manner without any risk of errors.

The conveyor is preferably a continuous or semi-continuous conveyor, and the package may or may not move through the storage unit in steps. When a package enters the storage unit, the packages that are already present in the storage unit are moved in downstream direction by the conveyor. In such a device, packages can be supplied to the infeed unit at a constant rate.

In one embodiment, the length of the conveyor and/or the speed of the conveyor is such that the package arrives at the discharge unit substantially at the same time as the result for the package in question is being displayed by the display means. The speed or average speed of a package is such that the time during which the package is present on the conveyor corresponds to, or is longer than, the time needed for analysing the sample. Said speed can be selected, based on the assay conditions of the sample analysis, such as the duration, before the device is put into operation. The analysis of the sample comprises adding reagents, allowing the sample with the reagents to stand for a predetermined period of time (contact time), and carrying out a measurement on the sample to test for the presence of microorganisms.

The conveyor is preferably configured so that, in use, the time during which a package stays in the conveyor is substantially equal to or longer than the duration of the analysis (contact time plus measurement time) of the sample from the package in question. The length of the conveyor divided by the speed or, in the case of movement in steps or of variable speed, the average speed of a package in the conveyor in use, can be set to be substantially equal to the duration of the analysis of the sample from the package in question.

In one embodiment of the device, the speed is adjustable, so that such a device can be easily adapted for different foodstuffs, wherein each foodstuff has its own specific analysis time different from that of the other foodstuffs. According to this embodiment, the time during which the package stays in the storage unit, in particular the conveyor, substantially depends on the duration of the analysis of the sample, and thus depends on the assay/protocol that is used. Different durations may be needed for different foodstuffs. For milk, for example, a contact time of about 15 minutes is needed, for fruit juices this is about 45 minutes. Using the present invention, the conveyor speed can be adapted in dependence on the foodstuffs being used and the associated protocol, such that the storage unit is used as efficiently as possible and the package arrives at the discharge end substantially at the same time as the sampling result. The device preferably comprises a control unit for controlling the conveyor speed. The device is thus suitable for a wide variety of foodstuffs.

In a preferred embodiment of the device according to the present invention, the storage unit comprises a multitude of parallel conveyor tracks. The parallel conveyor tracks are interconnected: a package on the first conveyor belt moves from the downstream end of a first conveyor belt to an end of a second conveyor belt, which second conveyor belt conveys the package further downstream. The conveyor tracks preferably have the same length. By making use of parallel conveyor tracks, a compact construction of the storage unit is realised.

In one embodiment, the parallel conveyor tracks are arranged substantially near each other and above or beside each other, wherein the directions of movement of the conveyor tracks alternate. The conveyor tracks are arranged above or beside each other in such a manner that there is sufficient space between adjacent conveyor tracks for a package to pass. The directions of movement of the conveyor tracks alternate, so that a package of a first conveyor track moves in the opposite direction of a package on a second conveyor track adjacent to the first conveyor track. If the conveyor tracks are arranged above each other, a lower conveyor track will move the package in forward direction, after which a conveyor track disposed above the lower conveyor track will move the package in rearward direction. The parallel conveyor tracks are configured to provide a substantially forward and backward movements of the package in the direction of the conveyor tracks as well as an effective movement of the package transversely to the direction of the conveyor tracks. Such an embodiment requires relatively little space because the package follows a relatively long path through a relatively small volume.

In one embodiment, the parallel conveyor tracks essentially form pairs of conveyor tracks, wherein the first conveyor track of each pair of conveyor tracks conveys a package in a first direction and a second conveyor track of the respective pair of conveyor tracks conveys the package in a second direction opposite the first direction. The conveyor tracks of one pair of conveyor tracks may be disposed near each other but also some distance apart. Thus it is possible to realise a square, spiral staircase-like embodiment, as it were.

In one embodiment, the conveyor further comprises a multitude of conveying means for transferring respective packages from one conveyor belt to a conveyor belt disposed therebeside or thereabove. The conveying means preferably convey a package in a direction substantially perpendicular to the conveying direction of the conveyor belts. The package can for example be transferred from a first, horizontal conveyor belt to a second, horizontal conveyor belt disposed above the first conveyor belt.

In one embodiment, the conveying directions of the multitude of conveyor tracks lie substantially in one plane of transport. The proportion between the length of the path of a package over the conveyor tracks and the surface area of the plane of transport that is defined by the conveying directions of the conveyor tracks is preferably substantially maximal, so that a package will move along the longest possible path, and will thus be present in the conveyor for the longest possible period of time, in relation to the area of the plane that is determined by the conveyor tracks.

In one embodiment, the conveyor further comprises additional conveyor tracks and additional conveying means, which lie at least substantially in one plane different from the plane of transport. Said additional conveyor tracks and additional conveying means may exhibit the same features and the same advantages as the conveyor tracks and conveying means described above.

The conveyor, and thus at least the parallel conveyor tracks and any further conveyor tracks, is configured so that a package can be removed from the storage unit at substantially any position within the storage unit. In one embodiment, every conveyor track is accessible from at least one side. The storage unit to that end comprises a frame, for example, to which the conveyor tracks are attached, in which frame openings are present, via which the package present on the conveyor tracks is accessible.

In one embodiment, the display means are configured to display information regarding the consumability of the foodstuff in the package. The result of the testing of the sample is transmitted to the display means from the analysis device. The display means preferably form part of the discharge unit, but they may be located remote from the discharge unit. Information regarding the result is displayed on the display means, so that this information is available to a user, for example, at the location of the discharge unit.

The display means are preferably configured to display the test results in a direct and clear manner. In one embodiment, the display means comprise at least one rejection indicator for displaying a result that corresponds to a tested amount of microorganisms in the sample above a specified upper limit value. An acceptance indicator will in that case be configured to display a result that corresponds to a tested amount of microorganisms in the sample below a specified lower limit value. The display means are for example provided with a red light and a green light, respectively indicating an undesirably high amount of microorganisms in the sample (and thus the package) and an amount of microorganisms suitable for consumption at a later point in time. Thus, the result of the sample can be linked to the package in question in a quick and effective manner, without any risk of errors, at the discharge unit. It is further conceivable for the display means to comprise additional indicators, for example an amber light for packages of which the testing result indicates that additional testing is required.

The device is preferably so compact that it can be used inside a sea container. The invention further relates to a sea container, in particular a sea container of the 2 TEU type, provided with a device according to the present invention. By providing the device inside a sea container, it can be readily be used as a mobile installation, so that the device can be used at various locations. The sea container is preferably disposed at the discharge end of a production line for packages containing foodstuffs, so that the packages can be tested for the presence of microorganisms in a simple manner.

The package is preferably stored inside the sea container during the analysis period. During said period, the package moves, possibly in steps, over a multitude of conveyor tracks that describe a winding path within the container such that the package will arrive at the display means in the sea container at the moment when the test results are being displayed by the display means. The conveyor tracks preferably extend above each other within the sea container, at least partially in the longitudinal direction of the sea container, such that the joint length of the multitude of conveyor tracks divided by the speed (or the average speed in the case of an interrupted movement) of the package over the multitude of conveyor tracks corresponds to the duration of the analysis of the sample. Preferably, at least two storage units according to the present invention are provided side by side in the sea container, so that a package can describe a relatively long path within the sea container without undergoing a relatively large effective movement. In addition to that, access to a package during said movement is possible when two storage units are used.

The invention further relates to a method for testing packaged foodstuffs for the presence of microorganisms, wherein a package containing a foodstuff is provided and a sample is taken of the foodstuff in the package, for example by means of a probe that extends into the interior of the package. The sample is tested for the presence of microorganisms and the results of the analysis of the sample are displayed. The method according to the present invention comprises the step of storing the package during said testing and the step of subsequently releasing the package, wherein the package is released substantially at the same time as the results of the package in question are being displayed at the location of the package. The moment the results of a package are shown, the package in question is released, so that a clear and simple link between the results and the package is made. The object of the present invention is thus achieved.

Preferably, the package is discharged from the device subsequently to being released.

The invention will now be explained in more detail with reference to a few embodiments as shown in the following figures. In the figures:
Figure 1a is a side view of a sea container comprising a device according to the present invention;
Figure 1b is a side view of the sea container of figure 1a, from the opposite side, with the device according to the present invention present therein;
Figure 2 is a cutaway top view of the device according to the present invention installed in a sea container as shown in figures 1a and 1b;
Figures 3a and 3b are a side view and a front view, respectively, of a storage unit for a device according to the present invention;
Figures 4a and 4b are schematic views of a discharge unit for a device according to the present invention.

In the first place it is noted that the invention will be explained hereinafter essentially with reference to an embodiment in which the device according to the present invention is installed in a sea container. The skilled person will appreciate, however, that the invention is not limited to placement of the device in a sea container. Other placements and uses are conceivable.

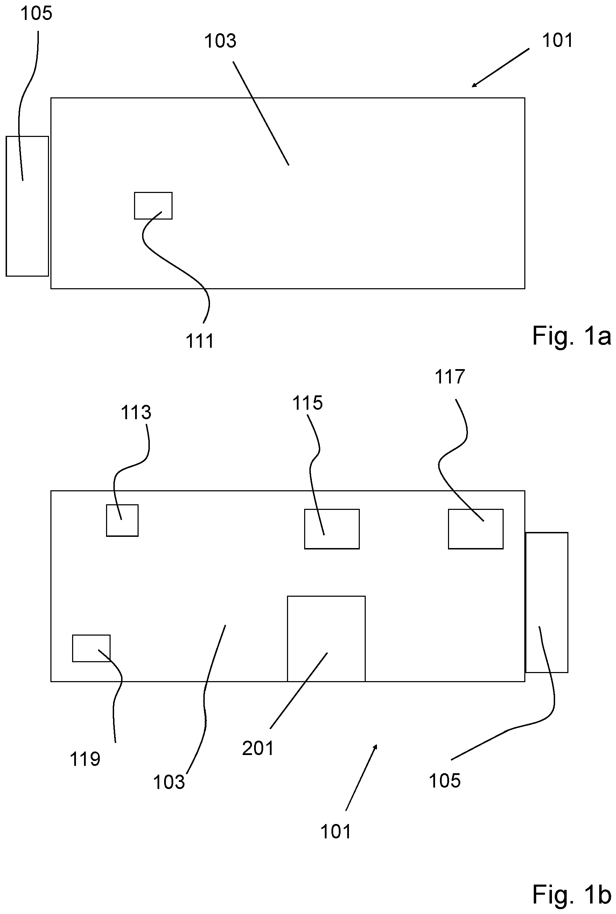

Figures 1a and 1b show a left-hand side view and a right-hand side view, respectively, of a sea container 101 comprising a container wall 103 and an access door 105, in which container a device according to the present invention for testing for the presence of microorganisms in packaged foodstuffs is provided. Provided in the side wall 103 of the container 101 are facilities and/or connections for the device, which are schematically shown in figures 1a and 1b. A water connection 111, an air outlet and/or inlet 113, a current connection 119 and an air conditioning connection 115, 107 are for example provided. Different connections may be provided, of course, depending on what is needed.

According to one aspect, the sea container is connected to a compressor unit 201. During use of the device, the compressor unit 201 is located outside the interior of the sea container 101, but the sea container 101 is arranged so that the compressor unit 201 can be accommodated in the interior of the sea container 101 during transport thereof. The device can thus be transported as an independent unit in a sea container and be set up locally without additional, or only minimal, means being required.

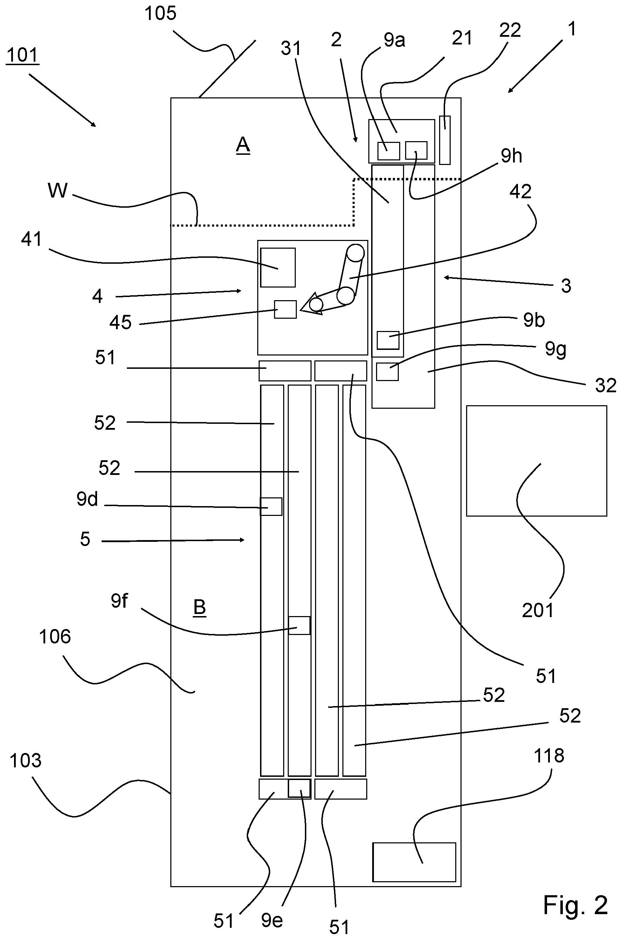

Figure 2 is a schematic top view of a sea container 101 provided with a device 1 according to the present invention. In particular the layout of the device 1 is schematically shown in this figure. In the illustrated embodiment, the interior of the sea container is divided into two parts A, B by means of a partition wall W. The two parts comprise an operator part A and a maintenance part B. During use, in particular the operator part A is in use and the maintenance part B is preferably closed for the sake of user safety. When the device 1 is not in use, the maintenance part is accessible, so that the equipment present therein can be cleaned, checked, repaired and/or maintained. Furthermore, a storage space (not shown) may be provided in the maintenance part B, for example for temporary storage of clothes, implements, reagents and/or samples. In one embodiment, the storage space comprises refrigerating means, so that reagents and/or samples can be safely stored.

The device 1 according to the present invention as shown in figure 2 is configured to test for the presence of microorganisms in packaged foodstuffs, in particular packages that have been selected or obtained as samples from a series of produced products. The device comprises an infeed unit 31 for a package containing a foodstuff that is to be analysed. In particular, a vibration device in the form of a shaker may be provided at the infeed unit 31, which device homogenises the foodstuff to be tested through vibration during a period of time and at a rate laid down in a predetermined protocol (assay). Provided downstream of the infeed unit 31 is a sample analysis device 4. The sample analysis device 4 is configured to take a sample from the foodstuff present in the package and also to test the sample for the presence of microorganisms therein. The device 1 further comprises a discharge unit 32, which is disposed downstream of the sample analysis device and which is configured to discharge the package from the device 1.

The operation of this sample analysis device 4 is known to the skilled person. For the sake of completeness the operation will be briefly explained. The sample analysis device 4 for example takes a sample from the package and transfers this to a "well" of so-called microtitre plate (for example 96-well plate). One or more reagents are added according to a predetermined protocol (assay) and subsequently, when all of the wells or a number of the wells are filled, the microtitre plate is placed on a vibrator (shaker) for a predetermined period of time that is needed for allowing said one or more reagents to act on the sample (exposure time). In the meantime, the package in question has already been carried forward and been stored, as will be explained in more detail yet. After the exposure time, a measurement can be carried out on the sample in the microtitre plate, and a result regarding the presence of microorganisms in the package in question can be provided.

In practice, several packages will be tested for the presence of microorganisms. These packages are successively supplied to the sample analysis device and a sample from each one of the packages is placed in the microtitre plate. The packages are transported further and stored.

When the microtitre plate is full, the sample analysis device will provide a new, empty microtitre plate, so that samples from further packages can be analysed.

According to the present invention, the device comprises a storage unit 5 for the package, which storage unit is disposed between the sample analysis device 4 and the discharge unit 32. That is, the storage unit is disposed downstream of the sample analysis device 4 and upstream of the discharge unit 32. The storage unit 5 is used for temporary storage of the package to be analysed, in particular for as long as the analysis takes, i.e. at least as long as the exposure time plus the time needed for the measurement. In the illustrated embodiment, the storage unit 5 is provided in the maintenance part B, such that the user does not have access to the package being analysed.

The device 1 according to the present invention comprises display means 22, which form part of an operator unit 2. Said display means 22 are configured to display a result of the testing of the sample from the package. The results are displayed in such a manner that they are perceptible, preferably visible or audible, at the location of the discharge unit. In one embodiment, the display means comprise a display screen. Additionally it is conceivable that an alternative signal is delivered in the case of a positive test result of the package.

In order to prevent any mix-up between results and packages, the device according to the present invention is configured so that the package is delivered at the end of the discharge unit 32 substantially at the same time as the results of the package in question are being displayed by the display means 22. As already mentioned before, a package will thus reach the end of the discharge unit 32 at the same time as its respective result (which is displayed by the display means), so that the result is linked to the associated package in an effective manner.

Further details of the embodiment shown in figure 2 will be explained hereinafter.

The operator unit 2 comprises a work table 21 and the aforesaid display means 22. The operator unit 2 is located in the operator part A. The infeed unit 31 and the discharge unit 32 connect to the operator unit and are in part located in the operator part, in such a manner that the operator can feed the package to be analysed into the device 1 and remove it therefrom. A package containing a foodstuff to be analysed can be placed on the infeed unit 31. Preferably, the package is placed in a container 9a suitable for that purpose, which is subsequently placed on the infeed unit 31.

The infeed unit 3 is configured to feed the package 9b to be analysed to the sample analysis device 4. In the illustrated embodiment, the sample analysis device 4 comprises a robot arm 42, which is configured to take a sample from a package 9b present at the sample analysis device 4, for example by taking a sample from the interior of the package, using a probe, and subsequently transferring it to an analysis device 41 for analysis thereof. The sample can for example be transferred in a microtitre plate 45, after which the samples present in the microtitre plate 45 are analysed in the analysis device 41, for example a luminometer (e.g. Promilite III made by Promicol B.V.). It is noted in that regard that the skilled person will in principle be familiar with the possible analyses to which samples can be subjected, in particular samples from the contents of a package, for example samples of milk or fruit juices. The invention is not limited to the type of analysis that is carried out; the sample analysis device 4 can in principle be used for carrying out any analysis that may be required.

Provided downstream of the sample analysis device 4 is the storage unit 5. After a sample has been taken, the package is carried to the storage unit 5. The storage unit 5 comprises four towers (in the illustrated embodiment) of a number of conveyors 52 arranged above each other. The conveyors 52 extend in the longitudinal direction of the sea container 101 and are placed substantially horizontally for transporting the containers 9d, 9f essentially forward and backward. Conveying means 51 are provided at both ends of the conveyors 52 for moving the containers 9e between the various conveyors 52. The conveying means may be configured to transfer respective packages from one conveyor belt 52 to a conveyor belt 52 disposed therebeside or thereabove.

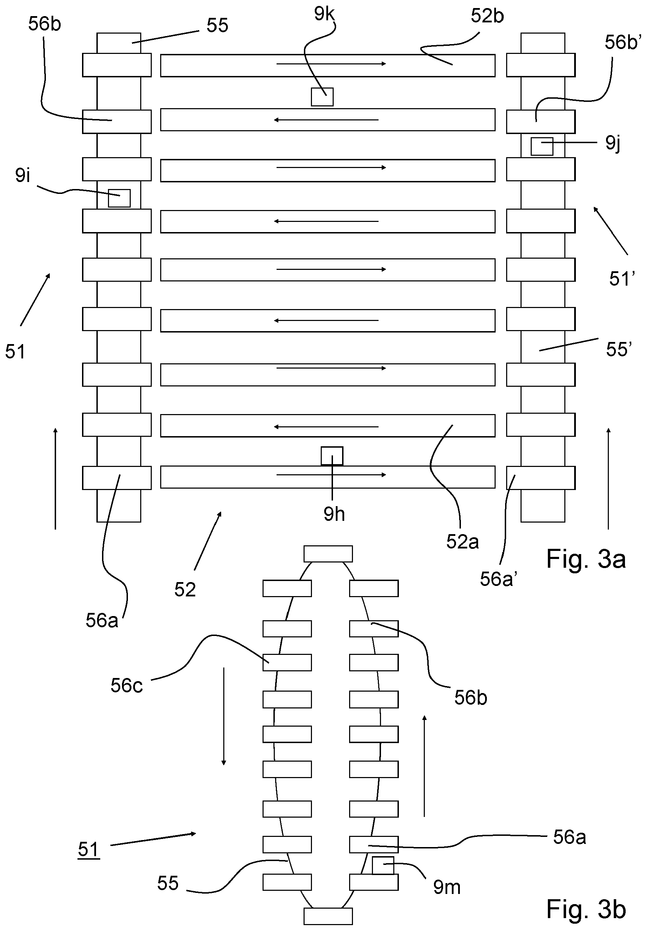

The storage unit 5 is shown in more detail in figures 3a and 3b, which schematically show a side view and a front view, respectively, of said storage unit 5. As shown in figure 3a, the storage unit 5 comprises nine conveyors 52a, 52b arranged above each other, which are alternately configured to move to the left (52a) or to the right (52b). Thus, parallel conveyor tracks 52a, 52b with alternate directions of movement are provided. Provided at the ends of the conveyor are the conveying means 51, 51' for moving the containers 9h, 9i, 9j between the conveyors 52a, 52b. Because the conveying means 51, 51' are embodied as rotating wheels (see figure 3b), the container 9m can also be moved between adjacent towers of conveyors (see also figure 2).

As a result of the configuration with the conveyors 52 and the aforesaid conveying means 51, the containers with packages can be stored for a long or a short period of time in the device, as desired, and that on a FIFO (first in first out) basis, such that when an analysis of a package is (nearly) done, this package can be transported to the discharge unit 32 again by means of the conveyor 52 in the storage unit 5, such that the result of the analysis can be transmitted to the display means 22, wherein the analysed package exits the discharge unit 32 substantially simultaneously therewith. By using a total of four towers of conveyors, it becomes possible to store an enormous amount of packages temporarily, which is advantageous in the case of tests that take relatively long. If desired, for example in the case of short tests, some of the towers need not be used. Alternatively, the speed of the conveyors can be increased or decreased, and that geared to the duration of the tests in such a manner that the analysed package will arrive at the end of the discharge unit 32 substantially simultaneously with the displayed result. The device is to that end preferably provided with a control unit 118 (see figure 2).

Because of the arrangement with four conveyor towers, the conveyor is accessible from the maintenance part B for removing a package from the conveyor at substantially any position within the conveyor. The conveyor will be readily accessible in case of a malfunction or if this is otherwise necessary, such that a desired container with a package (or a multitude of containers) can be removed from the conveyor.

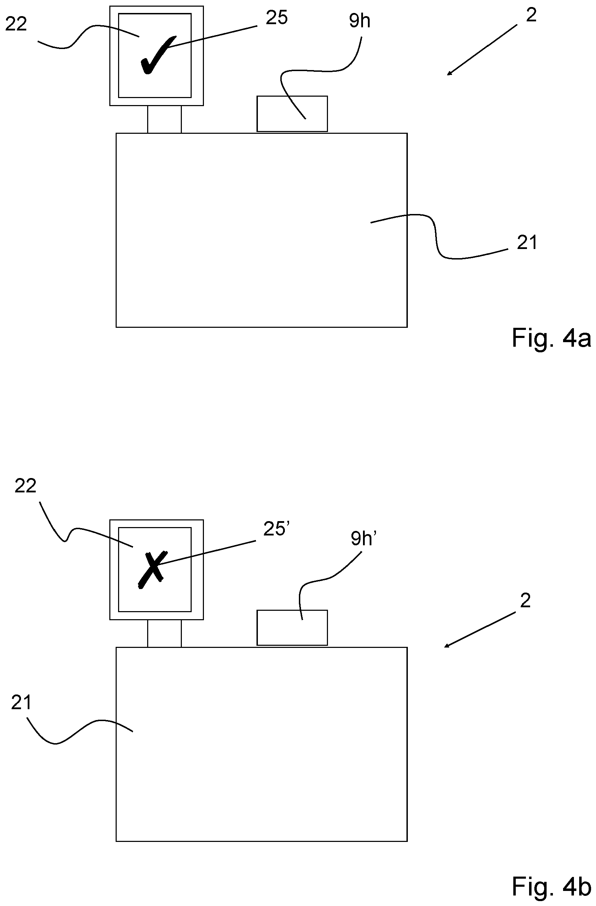

Referring to figures 4a and 4b, to conclude, the operator unit 2 with the work table 21 and the display means 22 is shown. The display means 22 are preferably configured to display information 25, 25' regarding the consumability of the foodstuff in the package 9h on the basis of the result from the analysis device 4. The information may comprise a rejection indicator 25' (figure 4b) for displaying a result that corresponds to a tested amount of microorganisms in the sample above a specified upper limit value, as well as an acceptance indicator 25 (figure 4a) for displaying a result that corresponds to a tested amount of microorganisms in the sample below a specified lower limit value. As already mentioned before, other information may be displayed. Furthermore, an audited signal may also be used in providing an acceptance indicator and/or a rejection indicator.

In the foregoing, the device 1 has been described on the basis of a situation in which the device is built into a sea container, in particular a sea container of the 2 TEU type. Although such an embodiment is advantageous, the invention is not limited thereto.

Using the device according to the present invention, testing packaged foodstuffs for the presence of microorganisms is very easy. According to the invention, a package containing a foodstuff is provided, for example near the infeed unit, a sample of the foodstuff in the package is taken, which sample is tested for the presence of microorganisms, for example using the sample analysis device, and the results of the analysis of the sample are displayed. According to the invention, the method comprises the step of storing the package during said testing and the step of subsequently releasing the package, wherein the package is released substantially at the same time as the results of the package in question are being displayed at the location of the package. The package can be stored in the storage unit. The package can be carried out of the device subsequent to being released.

In the foregoing, the invention has been explained with reference to a preferred embodiment, in which the device is provided in a sea container. The skilled person will appreciate, however, that although placement in a sea container is very advantageous, the device according to the present invention is not limited to placement in a sea container, but that a conventional setup, for example in a factory space or a laboratory space, is also conceivable. Many equivalent embodiments are conceivable within the scope of the invention. The protection sought is determined by the appended claims.

## Claims

1. A device (1) for testing packaged foodstuffs for the presence of microorganisms, the device comprising:
- an infeed unit (3) for a package containing a foodstuff;
- a sample analysis device (4) which is disposed downstream of the infeed unit and which is configured for taking a sample of the foodstuff in the package as well as for testing the sample for the presence of microorganisms;
- a discharge unit (32) which is disposed downstream of the sample analysis device for discharging the package from the device; wherein
- the device comprises a storage unit (5) for the package, which is disposed between the sample analysis device and the discharge unit;
- the device further comprises display means (22) for displaying a result of the testing of the sample from the package at the location of the discharge unit; and
- the device is configured so that the storage unit delivers the package at the discharge unit substantially at the same time as the results of the package in question are being displayed by the display means.

2. A device according to claim 1, wherein the storage unit comprises a conveyor (52), preferably the conveyor is configured to carry a multitude of packages into and out of the storage unit in the same order.

3. A device according to claim 2, wherein the conveyor is configured so that, in use, the time during which a package stays in the conveyor is substantially equal to the duration of the analysis of the sample from the package in question, preferably the length of the conveyor divided by the average speed of a package in the conveyor in use is substantially equal to the duration of the analysis of the sample from the package in question.

4. A device according to claim 2 or 3, wherein the length of the conveyor and/or the speed of the conveyor is selected or can be set so that the package arrives at the discharge unit substantially at the same time as the result for the package in question is being displayed by the display means, preferably the device comprises a control unit (118) for controlling the conveyor speed.

5. A device according to any one of claims 2 - 4, wherein the conveyor comprises a multitude of parallel conveyor tracks (52a, 52b), preferably the parallel conveyor tracks are arranged substantially near each other and above or beside each other, wherein the directions of movement of the conveyor tracks alternate, or the parallel conveyor tracks essentially form pairs of conveyor tracks, wherein the first conveyor track of each pair of conveyor tracks conveys a package in a first direction and a second conveyor track of the respective pair of conveyor tracks conveys the package in a second direction opposite the first direction.

6. A device according to claim 5, wherein the conveyor further comprises a multitude of conveying means (51, 51') for transferring respective packages from one conveyor belt to a conveyor belt disposed therebeside of thereabove, preferably the conveying means convey a package in a direction substantially perpendicular to the conveying direction of the conveyor belts.

7. A device according to claim 5 or 6, wherein the conveying directions of the multitude of conveyor tracks lie substantially in one plane of transport, preferably the proportion between the length of the path of a package over the conveyor tracks and the surface area of the plane of transport that is defined by the conveying directions of the conveyor tracks is substantially maximal.

8. A device according to claim 7, wherein the conveyor further comprises additional conveyor tracks and additional conveying means, which lie at least substantially in one plane different from the plane of transport.

9. A device according to claim 2 or 3, wherein the conveyor is configured to be accessible for removal of a package from the conveyor at substantially any position within the conveyor.

10. A device according to any one of the preceding claims, wherein the display means are configured to display information regarding the consumability of the foodstuff in the package on the basis of the result from the analysis device.

11. A device according to any one of the preceding claims, wherein the display means comprise at least one rejection indicator for displaying a result that corresponds to a tested amount of microorganisms in the sample above a specified upper limit value as well as an acceptance indicator for displaying a result that corresponds to a tested amount of microorganisms in the sample below a specified lower limit value.

12. A sea container (101), in particular a sea container of the 2 TEU type, comprising a device according to any one of the preceding claims.

13. A sea container according to claim 12, wherein the conveyor tracks extend above each other within the sea container, at least partially in the longitudinal direction of the sea container, such that the joint length of the multitude of conveyor tracks divided by the speed of the package over the multitude of conveyor tracks in use corresponds to the duration of the analysis of the sample.

14. A method for testing packaged foodstuffs for the presence of microorganisms by using a device according to any one of the claims 1-11 or a sea container according to claim 12 or 13, the method comprising the steps of:
- providing a package containing a foodstuff;
- taking a sample of the foodstuff in the package;
- testing the sample for the presence of microorganisms;
- displaying the result of the sample analysis,
**characterized in that** the method comprises the step of storing the package during said testing and the step of subsequently releasing the package, wherein the package is released substantially at the same time as the results of the package in question are displayed at the location of the package.

15. A method according to claim 14, wherein the package is discharged from the device subsequently to being released.

## Patentansprüche

1. Vorrichtung (1) zum Prüfen von verpackten Lebensmitteln auf das Vorhandensein von Mikroorganismen, wobei die Vorrichtung Folgendes umfasst:
- eine Zuführeinheit (3) für eine Packung, die ein Lebensmittel enthält;
- eine Probenanalysevorrichtung (4), die nach der Zuführeinheit angeordnet ist und zum Entnehmen einer Probe des Lebensmittels in der Packung sowie zum Prüfen der Probe auf das Vorhandensein von Mikroorganismen ausgelegt ist;
- eine Entladungseinheit (32), die nach der Probenanalysevorrichtung angeordnet ist und zum Entladen der Packung aus der Vorrichtung dient; wobei
- die Vorrichtung eine Lagereinheit (5) für die Packung umfasst, die zwischen der Probenanalysevorrichtung und der Entladungseinheit angeordnet ist;
- die Vorrichtung ferner Anzeigemittel (22) zum Anzeigen eines Ergebnisses der Prüfung der Probe aus der Packung an der Stelle der Austragseinheit umfasst; und
- die Vorrichtung so angeordnet ist, dass die Lagereinheit die Packung an die Entladungseinheit im Wesentlichen zum gleichen Zeitpunkt übergibt, an dem die Ergebnisse der betreffenden Packung durch die Anzeigemittel angezeigt werden.

2. Vorrichtung nach Anspruch 1, wobei die Lagereinheit eine Förderanlage (52) umfasst und die Förderanlage vorzugsweise so ausgelegt ist, dass sie eine Vielzahl von Packungen in der gleichen Reihenfolge in die Lagereinheit hinein und aus der Lagereinheit heraus transportiert.

3. Vorrichtung nach Anspruch 2, wobei die Förderanlage so ausgelegt ist, dass im Betrieb die Zeit, während der eine Packung in der Fördereinrichtung verbleibt, im Wesentlichen der Dauer der Analyse der Probe der betreffenden Packung entspricht, vorzugsweise ist die Länge der Förderanlage geteilt durch die durchschnittliche Geschwindigkeit einer Packung in der Fördereinrichtung im Betrieb im Wesentlichen gleich der Dauer der Analyse der Probe aus der betreffenden Packung.

4. Vorrichtung nach Anspruch 2 oder 3, wobei die Länge der Förderanlage und/oder die Geschwindigkeit der Förderanlage so ausgewählt oder eingestellt werden können, dass die Packung an der Entladungseinheit im Wesentlichen zur gleichen Zeit ankommt, zu der das Ergebnis für die betreffende Packung durch die Anzeigemittel angezeigt wird, vorzugsweise umfasst die Vorrichtung eine Steuereinheit (118) zum Steuern der Geschwindigkeit der Förderanlage.

5. Vorrichtung nach einem der Ansprüche 2-4, wobei die Förderanlage eine Vielzahl paralleler Förderbahnen (52a, 52b) umfasst und die parallelen Förderbahnen vorzugsweise nahe zueinander und über- oder nebeneinander angeordnet sind, wobei die Bewegungsrichtungen der Förderbahnen wechseln oder die parallelen Förderbahnen im Wesentlichen Paare von Förderbahnen ausbilden, bei denen die erste Förderbahn jedes Paars von Förderbahnen eine Packung in einer ersten Richtung transportiert und eine zweite Förderbahn des entsprechenden Paars von Förderbahnen die Packung in einer zweiten Richtung transportiert, die der ersten Richtung entgegengesetzt ist.

6. Vorrichtung nach Anspruch 5, wobei die Förderanlage ferner eine Vielzahl von Fördereinrichtungen (51, 51') zum Übertragen der entsprechenden Packungen von einem Förderband zu einem Förderband umfasst, das daneben oder darüber angeordnet ist, wobei vorzugsweise die Fördereinrichtungen eine Packung in einer Richtung transportieren, die im Wesentlichen senkrecht zur Förderrichtung der Förderbänder verläuft.

7. Vorrichtung nach Anspruch 5 oder 6, wobei die Förderrichtungen der Vielzahl der Förderbahnen im Wesentlichen in einer Transportebene liegen, vorzugsweise ist das Größenverhältnis zwischen der Länge des Wegs einer Packung über die Förderbahnen und der Oberfläche der Transportebene, die durch die Förderrichtungen der Förderbahnen definiert wird, im Wesentlichen maximal.

8. Vorrichtung nach Anspruch 7, wobei die Förderanlage ferner zusätzliche Förderbahnen und zusätzliche Fördermittel umfasst, die mindestens im Wesentlichen in einer Ebene liegen, die sich von der Transportebene unterscheidet.

9. Vorrichtung nach Anspruch 2 oder 3, wobei die Förderanlage so ausgelegt ist, dass sie zum Entnehmen einer Packung aus der Förderanlage an im Wesentlichen jeder beliebigen Stelle innerhalb der Förderanlage zugänglich ist.

10. Vorrichtung nach einem der vorangehenden Ansprüche, wobei die Anzeigemittel so ausgelegt sind, dass sie die Informationen zur Verzehrbarkeit des Lebensmittels in der Packung anhand des Ergebnisses der Analysevorrichtung anzeigen.

11. Vorrichtung nach einem der vorangehenden Ansprüche, wobei die Anzeigemittel mindestens einen Ablehnungsindikator zum Anzeigen eines Ergebnisses, das einer geprüften Menge an Mikroorganismen in der Probe oberhalb eines vorgegebenen oberen Grenzwerts entspricht, sowie einen Abnahmeindikator zum Anzeigen eines Ergebnisses umfassen, das einer geprüften Menge an Mikroorganismen in der Probe unterhalb eines vorgegebenen oberen Grenzwerts entspricht.

12. Seecontainer (101), insbesondere ein Seecontainer des Typs 2 TEU, der eine Vorrichtung nach einem der vorangehenden Ansprüche umfasst.

13. Seecontainer nach Anspruch 12, wobei sich die Förderbahnen übereinander innerhalb des Seecontainers erstrecken, mindestens teilweise in Längsrichtung des Seecontainers, so dass die gemeinsame Länge der Vielzahl der Förderbahnen geteilt durch die Geschwindigkeit der Packung über die in Betrieb befindliche Menge der Förderbahnen der Dauer der Analyse der Probe entspricht.

14. Verfahren zum Prüfen verpackter Lebensmittel auf das Vorhandensein von Mikroorganismen unter Verwendung einer Vorrichtung nach einem der Ansprüche 1-11 oder eines Seecontainers nach Anspruch 12 oder 13, wobei das Verfahren die folgenden Schritte umfasst:
- Bereitstellen einer Packung, die ein Lebensmittel enthält;
- Entnehmen einer Probe des Lebensmittels in der Packung;
- Prüfen der Probe auf das Vorhandensein von Mikroorganismen;
- Anzeigen des Ergebnisses der Probenanalyse,
**dadurch gekennzeichnet, dass** das Verfahren den Schritt der Lagerung der Packung während des Prüfens und den Schritt der nachfolgenden Freigabe der Packung umfasst, wobei die Packung im Wesentlichen zur gleichen Zeit freigegeben wird, wie die Ergebnisse der betreffenden Packung am Standort der Packung angezeigt werden.

15. Verfahren nach Anspruch 14, wobei die Packung nach der Freigabe aus der Vorrichtung entladen wird.

## Revendications

1. Dispositif (1) pour tester la présence de microorganismes dans des denrées alimentaires emballées, le dispositif comprenant:
- une unité d'alimentation (3) pour un emballage contenant une denrée alimentaire;
- un dispositif d'analyse d'échantillon (4) qui est disposé en aval de l'unité d'alimentation et qui est configuré pour prélever un échantillon de la denrée alimentaire dans l'emballage ainsi que pour tester la présence de microorganismes dans l'échantillon;
- une unité d'évacuation (32) qui est disposée en aval du dispositif d'analyse d'échantillon pour évacuer l'emballage du dispositif;
dans lequel
- le dispositif comprend une unité de stockage (5) pour l'emballage, qui est disposée entre le dispositif d'analyse d'échantillon et l'unité d'évacuation ;
- le dispositif comprend en outre des moyens d'affichage (22) pour afficher un résultat du test de l'échantillon provenant de l'emballage à l'emplacement de l'unité d'évacuation; et
- le dispositif est configuré de sorte que l'unité de stockage distribue l'emballage au niveau de l'unité d'évacuation essentiellement en même temps que les résultats de l'emballage en question sont affichés par les moyens d'affichage.

2. Dispositif selon la revendication 1, dans lequel l'unité de stockage comprend un transporteur (52), de préférence, le transporteur est configuré pour porter une multitude d'emballages dans et hors de l'unité de stockage dans le même ordre.

3. Dispositif selon la revendication 2, dans lequel le transporteur est configuré de sorte que, en cours d'utilisation, le temps pendant lequel un emballage reste dans le transporteur soit essentiellement égal à la durée de l'analyse de l'échantillon provenant de l'emballage en question, de préférence la longueur du transporteur divisée par la vitesse moyenne d'un emballage dans le transporteur en cours d'utilisation est essentiellement égale à la durée de l'analyse de l'échantillon provenant de l'emballage en question.

4. Dispositif selon la revendication 2 ou 3, dans lequel la longueur du transporteur et/ou la vitesse du transporteur est/sont choisie(s) ou peut/peuvent être définie(s) de sorte que l'emballage arrive au niveau de l'unité d'évacuation essentiellement en même temps que le résultat de l'emballage en question est affiché par les moyens d'affichage, de préférence le dispositif comprend une unité de commande (118) pour commander la vitesse de transporteur.

5. Dispositif selon l'une quelconque des revendications 2 à 4, dans lequel le transporteur comprend une multitude de voies de transport parallèles (52a, 52b), de préférence les voies de transport parallèles sont agencées essentiellement les unes près des autres et les unes au-dessus des autres ou les unes à côté des autres, où les directions de déplacement des voies de transport sont alternées, ou les voies de transport parallèles forment essentiellement des paires de voies de transport, où la première voie de transport de chaque paire de voies de transport transporte un emballage dans une première direction et une deuxième voie de transport de la paire respective de voies de transport transporte l'emballage dans une deuxième direction opposée à la première direction.

6. Dispositif selon la revendication 5, dans lequel le transporteur comprend en outre une multitude de moyens de transport (51, 51') pour transférer des emballages respectifs d'une bande transporteuse à une bande transporteuse disposée à côté de celle-ci ou au-dessus de celle-ci, de préférence les moyens de transport transportent un emballage dans une direction essentiellement perpendiculaire à la direction de transport des bandes transporteuses.

7. Dispositif selon la revendication 5 ou 6, dans lequel les directions de transport de la multitude de voies de transport se trouvent essentiellement dans un plan de transport, de préférence la proportion entre la longueur du chemin d'un emballage sur les voies de transport et la surface du plan de transport qui est défini par les directions de transport des voies de transport est essentiellement maximale.

8. Dispositif selon la revendication 7, dans lequel le transporteur comprend en outre des voies de transport supplémentaires et des moyens de transport supplémentaires, qui se trouvent au moins essentiellement dans un plan différent du plan de transport.

9. Dispositif selon la revendication 2 ou 3, dans lequel le transporteur est configuré pour être accessible pour le retrait d'un emballage du transporteur à essentiellement n'importe quelle position dans le transporteur.

10. Dispositif selon l'une quelconque des revendications précédentes, dans lequel les moyens d'affichage sont configurés pour afficher des informations concernant la consommabilité de la denrée alimentaire dans l'emballage sur la base du résultat du dispositif d'analyse.

11. Dispositif selon l'une quelconque des revendications précédentes, dans lequel les moyens d'affichage comprennent au moins un indicateur de rejet pour afficher un résultat qui correspond à une quantité testée de microorganismes dans l'échantillon au-dessus d'une valeur limite supérieure spécifiée ainsi qu'un indicateur d'acceptation pour afficher un résultat qui correspond à une quantité testée de microorganismes dans l'échantillon en dessous d'une valeur limite inférieure spécifiée.

12. Conteneur maritime (101), en particulier un conteneur maritime du type 2 TEU, comprenant un dispositif selon l'une quelconque des revendications précédentes.

13. Conteneur maritime selon la revendication 12, dans lequel les voies de transport s'étendent les unes au-dessus des autres dans le conteneur maritime, au moins partiellement dans la direction longitudinale du conteneur maritime, de sorte que la longueur commune de la multitude de voies de transport divisée par la vitesse de l'emballage sur la multitude de voies de transport en cours d'utilisation corresponde à la durée de l'analyse de l'échantillon.

14. Procédé pour tester la présence de microorganismes dans des denrées alimentaires emballées en utilisant un dispositif selon l'une quelconque des revendications 1 à 11 ou un conteneur maritime selon la revendication 12 ou 13, le procédé comprenant les étapes consistant:
- à fournir un emballage contenant une denrée alimentaire;
- à prélever un échantillon de la denrée alimentaire dans l'emballage;
- à tester la présence de microorganismes dans l'échantillon;
- à afficher le résultat de l'analyse d'échantillon,
**caractérisé en ce que** le procédé comprend l'étape consistant à stocker l'emballage pendant ledit test et l'étape consistant à libérer par la suite l'emballage, où l'emballage est libéré essentiellement en même temps que les résultats de l'emballage en question sont affichés à l'emplacement de l'emballage.

15. Procédé selon la revendication 14, dans lequel l'emballage est évacué du dispositif après sa libération.
